# EUROPÄISCHE PATENTSCHRIFT

(11) **EP 0 929 320 B1**
(45) Veröffentlichungstag und Bekanntmachung des Hinweises auf die Patenterteilung: **18.07.2001**
(21) Anmeldenummer: 97910223.3
(22) Anmeldetag: 25.09.1997
(51) Int. Cl.: A61L 2/24, C02F 1/68

(54) **DOSIERVORRICHTUNG ZUR ZUGABE VON ENTKEIMUNGS- ODER DESINFEKTIONSMITTEL IN EINE WASSERGESPEISTE VERSORGUNGSEINRICHTUNG SOWIE DEREN VERWENDUNG**
DOSING MECHANISM TO FEED GERMICIDE OR DISINFECTANT INTO A WATER SUPPLIED SUPPLY DEVICE AND THE USE THEREOF
DISPOSITIF DE DOSAGE POUR DELIVRER UN AGENT GERMICIDE OU DESINFECTANT DANS UN DISPOSITIF D'ALIMENTATION ALIMENTE EN EAU ET SON UTILISATION

(30) Priorität: 27.09.1996 DE 19639666
(43) Veröffentlichungstag der Anmeldung: 21.07.1999
(73) Patentinhaber: Alpro Dental-Produkte GmbH, 78112 St. Georgen (DE)
(72) Erfinder: RUPPENTHAL, Mathias, D-76646 Bruchsal (DE)
(74) Vertreter: Patentanwälte Westphal, Mussgnug & Partner
(86) Internationale Anmeldenummer: DE9702190
(87) Internationale Veröffentlichungsnummer: WO9813074

(56) Entgegenhaltungen:
- EP-A- 0 111 249
- WO-A-95/20366
- DE-A- 3 403 640
- DE-A- 3 413 726

## Beschreibung

Die Erfindung betrifft eine Dosiervorrichtung zur Zugabe von Entkeimungs- oder Desinfektionsmittel in eine wassergespeiste Versorgungseinrichtung zur Bereitstellung von entkeimtem/ desinfiziertem Wasser für Verbraucher gemäß dem Oberbegriff des Anspruchs 1 sowie deren Verwendung.

Es ist allgemein bekannt, Wasser durch Zugabe bestimmter chemischer Substanzen keimfrei zu machen. Dabei ist es üblich, das Entkeimungs- oder Desinfektionsmittel in Tablettenform oder, bei größeren Mengen, als Lösung der zu entkeimenden Wassermenge zuzusetzen, um so eine bessere Durchmischung des zu entkeimenden Wassers zu gewährleisten. Unabhängig von der Art der Zugabe ist dabei für die gewünschte Entkeimung oder Desinfektion darauf zu achten, daß eine gute Durchmischung des Wassers mit dem zugegebenen Entkeimungs- oder Desinfektionsmittel erfolgt.

Ein verbreiteter Anwendungsfall ist die Entkeimung des bei der Dentalhygiene in zahnärztlichen Praxen für die Patienten benötigten Wassers, zum Beispiel zum Ausspülen des Mundes.

Üblicherweise kommen hierbei Dosiervorrichtungen zur Anwendung, bei denen das Wasser periodisch geimpft wird. Dies bedeutet, daß das Entkeimungsmittel zyklisch zugegeben wird, da diese Vorrichtungen überwiegend mit getakteten Magneten arbeiten, die in hierbei eingesetzten Pumpen und/oder Ventilen vorgesehen sind.

Die derartigen Komponenten erlauben nur große Schaltintervalle, so daß auch die Intervalle zwischen den einzelnen Impfungen entsprechend groß sind. Erhebliche Schwankungen der Konzentration von Entkeimungsmittel im Wasser sind die Folge.

Eine weitere bekannte Vorrichtung besitzt einen mit Druckluft beaufschlagten Vorratsbehälter für Entkeimungsmittel, der über eine entsprechende Rohr- oder Schlauchleitung mit einem Verbraucher verbunden ist. In diesem Leitungsweg ist ein fernbetätigbares Zulaufventil für das Entkeimungsmittel angeordnet, welches die Einleitung des Entkeimungsmittels in den Strömungsweg für das Wasser steuert, welcher zu dem Verbraucher führt. Bei Aktivierung des Verbrauchers öffnet dieses Zulaufventil und in Pausenzeiten ist es geschlossen.

Eine besondere Mengenerfassung des Wassers ist bei dieser Einrichtung nicht vorgesehen. Die Zumischung des Entkeimungsmittels erfolgt mit Hilfe eines getakteten Magnetventils, dessen Öffnung wiederum zyklisch erfolgt, sobald der Verbraucher, zum Beispiel Wasserauslaß für ein Mundspülglas, eingeschaltet wird, das heißt, sobald dessen Auslaß aktiviert wird. Konzentrationsschwankungen sind auch hier unvermeidbar.

Schließlich ist aus der DE 34 03 640 A1 eine Dosiervorrichtung bekannt, von der die Erfindung ausgeht. Bei dieser Vorrichtung wird das Entkeimungsmittel dem Wasser in einer speziellen Mischkammer zugeführt, die in eine Versorgungsleitung eingebaut ist. In diese Mischkammer mündet die Druckleitung einer Kolbenpumpe, die das Entkeimungsmittel fördert.

Zum Zweck des Dosierens ist eine Steuereinrichtung vorhanden, die in Abhängigkeit der Strömungsmenge bzw. Strömungsgeschwindigkeit in der Versorgungsleitung auf die Kolbenpumpe einwirkt, so daß die gewünschte Menge an Entkeimungsmittel aus dem Vorratsbehälter gefördert wird. Die Einwirkung auf die Kolbenpumpe kann entweder über die Drehzahl des Antriebsmotors oder das Übersetzungsverhältnis des Untersetzungsgetriebes erfolgen, um den Druckhub der Pumpe bedarfsgerecht anzupassen.

Als nachteilig wird hierbei empfunden, daß die desinfizierende Wirkung des Entkeimungsmittels in ausreichendem Umfang lediglich in der Mischkammer selbst gegeben ist. Bei längeren Standzeiten, wie sie beispielsweise im Dentalbereich zwischen zwei aufeinanderfolgenden Tagen an Wochenenden üblich sind, besteht deshalb die Gefahr, daß die desinfizierende Wirkung abnimmt und unzulässig hohe Kontaminationswerte auftreten. Weiterhin tritt das Problem auf, daß sich im Laufe des üblichen Gebrauchs abgetötete Bakterien und sonstige organische Substanzen als sogenannter Biofilm im Bereich der Versorgungsleitung und in den nachgeschalteten wasserführenden Wegen anlagern, die als idealer Nährboden für weitere Keime gilt. Es ist deshalb erforderlich, in gewissen Zeiten eine vollständige Entkeimung (Grund- oder Intensiventkeimung) vorzunehmen.

Zu diesem Zweck muß die Vorrichtung stillgesetzt werden. Danach werden sämtliche Schlauchverbindungen gelöst und es erfolgt der Anschluß an ein separates Pumpensystem, das speziell für die Vollentkeimung vorgesehen ist und die Behandlung mit einer hundertprozentigen Konzentration an Entkeimungsmittel zuläßt mit der Zielsetzung, den Biofilm zu entfernen und die wasserführenden Elemente zu sanieren. Im Anschluß daran muß das Leitungssystem mit reinem Wasser gespült werden, um beim darauffolgenden Behandlungsbeginn eine unzulässig hohe Konzentration von Entkeimungsmittel zu vermeiden. Ein derartiger Reinigungsvorgang ist äußerst zeit- und kostenaufwendig.

Auch ist der konstruktive Aufwand dieser Vorrichtung erheblich, da neben einer in die Versorgungsleitung einzusetzenden Mischkammer insbesondere die verwendete Kolbenpumpe einschließlich deren Ansteuerung relativ kompliziert ist. Neben der Notwendigkeit zur Beeinflussung der Drehzahl und/oder des Untersetzungsverhältnisses des Getriebes müssen spezielle Vorkehrungen in Form von Überdruckventilen und Rücklaufleitungen vorgesehen werden, um die Funktionssicherheit zu gewährleisten.

Schließlich tritt auch bei dieser Vorrichtung eine unerwünschte Konzentrationsschwankung von Entkeimungsmittel im Wasser auf, da die Kolbenpumpe das Entkeimungsmittel entsprechend dem Hubvolumen der Kolbenpumpe zyklisch zuführt.

Ausgehend von diesem Stand der Technik ist es Aufgabe der Erfindung, eine Dosiereinrichtung der eingangs genannten Art zu schaffen, welche einfach gestaltet und leicht handhabbar ist und eine bedarfsgerechte Zugabe von Entkeimungsmittel gewährleistet.

Diese Aufgabe wird erfindungsgemäß durch die Merkmale des Patentanspruchs 1 gelöst. Weitere vorteilhafte Ausgestaltungen und deren Merkmale sind in den Unteransprüchen angegeben.

Die Erfindung basiert auf der Idee, auf eine Pumpe einschließlich ihrer komplizierten Ansteuerung vollständig zu verzichten und statt dessen einen einfachen, mit Druckluft beaufschlagbaren Vorratsbehälter vorzusehen und die Zugabe von Entkeimungs-/Desinfektionsmittel über ein von der Steuerungseinrichtung ansteuerbares Proportionalventil zu realisieren. Neben der damit verbundenen Vereinfachung des Aufbaus ergibt sich insbesondere der Vorteil einer kontinuierlichen und völlig gleichmäßigen Zugabe von Entkeimungs-/Desinfektionsmittel in das vorbeiströmende Wasser. Konzentrationsschwankungen, wie sie bei allen vorbekannten Dosiereinrichtungen als unvermeidbar hingenommen wurden, treten hier nicht mehr auf. Damit läßt sich eine sichere Entkeimung mit reduzierter Menge an Entkeimungs-/Desinfektionsmittel erzielen.

Der erforderliche Förderdruck, mit dem der Vorratsbehälter zu beaufschlagen ist, kann mit geringstem Aufwand aus dem Umfeld gewonnen werden, in welchem derartige Dosiervorrichtungen betrieben werden. Als Beispiel kann hierbei der Druckluftanschluß für den Antrieb einer Bohrerturbine einer Dentaleinheit gelten.

Darüber hinaus kann auf eine Mischkammer vollständig verzichtet werden, da das Entkeimungsmittel gewünschtenfalls unmittelbar in die Zulaufleitung über das Proportionalventil zufließen kann. Wegen des geringen Platzbedarfs des Proportionalventils ist es möglich, die Zulaufstelle unmittelbar benachbart zur Abgabestelle vorzusehen, so daß eine Kontamination auch bei längeren Stillstandszeiten kaum zu beobachten ist.

Bevorzugt wird als Proportionalventil ein Piezoventil eingesetzt, welches besonders kompakt aufgebaut und zudem zuverlässig ansteuerbar ist.

Gemäß einer weiteren bevorzugten Ausführungsform der Erfindung ist durch die Steuerungseinrichtung das Proportionalventil so ansteuerbar, daß die Konzentration an Entkeimungsmittel variabel zwischen 0 % und 100 % einstellbar ist. Damit ist es möglich, die von Zeit zu Zeit erforderliche Intensiventkeimung ohne besondere, zusätzliche Maßnahme durchzuführen. Das Proportionalventil wird in Verbindung mit dem Zulaufventil für das Wasser so angesteuert, daß der Wasserzufluß vollständig gesperrt und ausschließlich Entkeimungsmittel zufließen kann. Für den sich anschließenden Spülvorgang muß lediglich der Zufluß des Entkeimungsmittels zurückgenommen werden, so daß reines Wasser zufließen kann. Die Beschaltung dieser Betriebszustände erfolgt durch die Steuerungseinrichtung, wobei keine zusätzlichen Bauteile oder Umbaumaßnahmen erforderlich sind.

Gemäß einer weiteren bevorzugten Ausführungsform ist eine Einrichtung zur Erfassung des IST-Wertes der Konzentration nach der Zugabestelle vorgesehen, die mit der Steuerungseinrichtung zu einem geschlossenen Regelkreis verbunden ist. Damit kann kontinuierlich die tatsächlich erreichte Konzentration überwacht und auf einem konstanten Wert präzise eingehalten werden.

Weiterhin ist die Dosiervorrichtung bevorzugt mit einer Einrichtung zur Selbstkalibrierung versehen, um auch bei längerer Gebrauchsdauer Alterungserescheinungen oder Veränderungen im Ansprechverhalten des Proportionalventils entgegenzuwirken. Es kann vorgesehen sein, die Selbstkalibrierung in gewissen, fest vorgegebenen Zeitabständen oder aber auch nach längeren Stillstandszeiten automatisch ablaufen zu lassen.

Entsprechend einer weiteren Ausgestaltung der Erfindung ist die Einleitung des Entkeimungsmittels in den Strömungsweg des eingespeisten Wassers vor dem Durchflußmesser vorgesehen.

Hierbei wird vorteilhafterweise ausgenutzt, daß der Durchflußmesser wenigstens einen Drehkörper aufweist, zum Beispiel ein Flügelrad, welches das eingespeiste Wasser und das eingeleitete Entkeimungsmittel durcheinanderwirbelt und hierdurch innig miteinander vermischt.

Bei einer zweckmäßigen Ausführungsform der Erfindung ist die Einleitung des Entkeimungsmittels in den Strömungsweg des eingespeisten Wassers in der Nähe zu einem Verbraucher vorgesehen.

Gemäß einer weiteren bevorzugten Ausgestaltung ist zwischen der Stelle der Einleitung des Entkeimungsmittels in den Strömungsweg des Wassers und dem Vorratsbehälter ein Rückschlagventil angeordnet, welches den Zutritt von Wasser in den Vorratsbehälter sicher ausschließt. Ferner kann vorteilhafterweise zwischen der Stelle der Einleitung des Entkeimungsmittels in den Strömungsweg des Wassers und dem Vorratsbehälter auch eine Filtereinrichtung angeordnet sein, welche den Zutritt von Verunreinigungen und/oder Verklumpungen aus dem Vorratsbehälter, welche die Vermischung und Löslichkeit im Wasser beeinträchtigen können, sicher ausschließt.

Für den Fall, daß eine über das übliche Maß hinausgehende Menge an Entkeimungsmittel benötigt wird, kann für die Einleitung des Entkeimungsmittels in den Strömungsweg des Wassers eine zweite Leitung mit einem zweiten Zulaufventil vorgesehen sein, welches parallel zum ersten Zulaufventil arbeitet, und für eine raschere Einleitung des Entkeimungsmittels in einer zweiten Einleitungsstelle sorgt, die beispielsweise stromab der ersten Einleitungsstelle angeordnet ist.

Weitere bevorzugte Varianten beziehen sich auf die Konzeption eines intergrierten Pufferspeichers für entkeimtes Wasser. So kann es bei stark keimbelastetem Wasser erforderlich sein, die Einwirkzeit des zudosierten Entkeimungsmittels zu verlängern. Je nach Keimart ist eine unterschiedlich lange Einwirkzeit des Entkeimungsmittels erforderlich. Der Pufferspeicher sichert auch im Dauerbetrieb die Einhaltung der notwendigen Einwirkzeit zwischen Zudosierung und Austritt am Instrumentenende. Eine Möglichkeit hierfür bietet der Pufferspeicher, der die Verweilzeit des mit Entkeimungsmittel versetzten Wassers verlängert.

Bevorzugt ist der Pufferspeicher aus einem Rohrpaket aufbaut, das beispielsweise aus einer Vielzahl nebeneinanderliegender flacher Rohre besteht. Besonders platzsparend ist ein Bündel von Rohren mit rechteckigem oder quadratischem Querschnitt, so daß der Strömungsquerschnitt optimal genutzt wird. Das Rohrpaket ist zur Bildung eines durchgehenden Strömungspfades jeweils stirnseitig mit Endstücken verschlossen, wobei die Endstücke Aushöhlungen derart aufweisen, daß eine fluiddichte, jeweils paarweise Verbindung zweier benachbarter Rohre entsteht.

Bevorzugt ist der Pufferspeicher in ein Aufnahmegehäuse eingesetzt, so daß ein Zusatzmodul entsteht, welches entweder unmittelbar am Gehäuse der Dosiervorrichtung oder aber der Dosiervorrichtung nachgeschaltet anbringbar ist.

In vorteilhafter Weise ist die erfindungsgemäße Dosiereinrichtung als Zusatzmodul ausgebildet, welches nachträglich in eine bestehende Geräteanordnung für die Dentalhygiene integrierbar ist.

Bevorzugt ist die erfindungsgemäße Dosiervorrichtung zur Verwendung für Einrichtungen der Dentalhygiene, zum Beispiel in der zahnärztlichen Praxis an Dentaleinheiten, vorgesehen. Anders als bei bekannten Einrichtungen dieser Art, beruht die Bestimmung der Menge des Entkeimungsmittels für die Einleitung ausschließlich auf der Durchflußmenge an Wasser. Hieraus folgt, daß die Kenntnis weiterer Kenngrößen, zum Beispiel der Umgebung und der Drücke, in der die erfindungsgemäße Dosiereinrichtung zum Einsatz kommt, für deren Funktion nicht erforderlich ist. Die Nachrüstung vorhandener Dentaleinheiten ohne Entkeimung ist damit kostengünstig möglich. Ebenso eignet sich die erfindungsgemäße Dosiervorrichtung auch zur Zudosierung vergleichbarer Substanzen, wie beispielsweise von Reinigungsmitteln.

Anhand eines schematisch dargestellten Ausführungsbeispiels der Erfindung sollen die Erfindung, vorteilhafte Ausgestaltungen und Verbesserungen sowie besondere Vorteile näher erläutert und beschrieben werden. Es zeigen:
- Fig. 1: Schemaskizze für eine Schaltungsanordnung einer erfindungsgemäßen Dosiervorrichtung;
- Fig. 2: Seitenansicht einer erfindungsgemäßen Dosiervorrichtung;
- Fig. 3: Draufsicht auf die Dosiervorrichtung gemäß Fig. 2,
- Fig. 4: Stirnansicht der erfindungsgemäßen Dosiervorrichtung gemäß Fig. 2,
- Fig. 5: Pufferspeicher als Zusatzmodul,
- Fig. 6: Dosiervorrichtung mit Pufferspeicher.

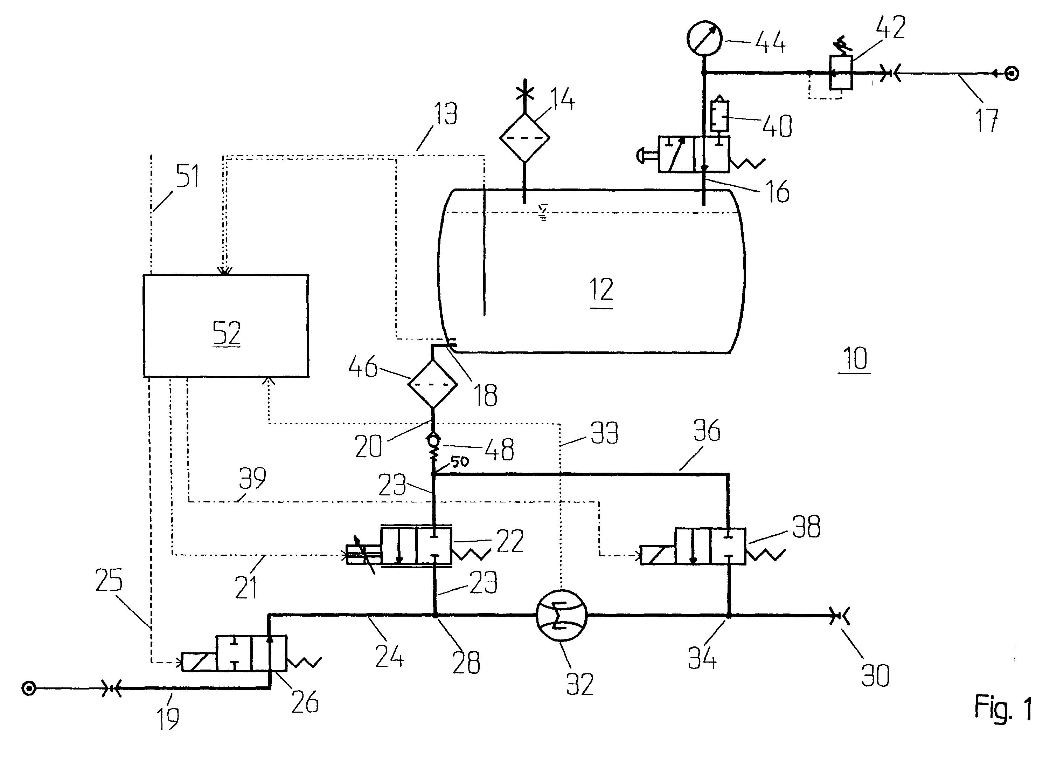

In Fig. 1 ist eine Dosiervorrichtung 10 mit einem Vorratsbehälter 12 für flüssiges Entkeimungsmittel dargestellt, wobei der Vorratsbehälter 12 an seiner Oberseite einen hier nur angedeuteten Einfüllstutzen mit integriertem Sieb 14 aufweist, durch welchen die Befüllung des Vorratsbehälters 12 mit Entkeimungsmittel erfolgt.

Ferner besitzt der Vorratsbehälter 12 an seiner Oberseite einen Anschlußstutzen 16 für eine Druckluftleitung 17, mittels welcher der Vorratsbehälter 12 unter Innendruck gesetzt wird. Hierdurch ist gewährleistet, daß das Entkeimungsmittel stetig aus dem Vorratsbehälter 12 ausströmen kann.

Zu diesem Zweck ist an der einen Stirnseite, in der Fig. 1 die linke Stirnseite, ein Auslaßstutzen 18 angeordnet, der mit einer Zulaufleitung 20 verbunden ist, die zu einem ersten, als Proportionsventil ausgebildetes Zulaufventil 22 führt. Dieses erste Zulaufventil 22 ist als elektrisch betätigbares Proportionalventil ausgebildet und dient dazu, die Menge an Entkeimungsmittel zur Einleitung über eine erste Zulaufleitung 23 in eine Wasserleitung 24 einzustellen.

Die Wasserleitung 24, in welcher ebenfalls ein elektrisch betätigbares Ventil 26 angeordnet ist, das den Wasserzulauf steuert, führt von einem nicht näher gezeigten Versorgungsnetz zu einer Anschlußstelle 30 für einen oder mehrere, hier ebenfalls nicht näher gezeigte Verbraucher.

In dem in Fig. 1 gezeigten Leitungsabschnitt der Wasserleitung 24 befinden sich zunächst eine erste Einleitungsstelle 28 für die erste Zulaufleitung 23 für Entkeimungsmittel.

Stromabwärts dieser Einleitungsstelle 28 ist ein Durchflußmeßgerät 32 angeordnet, das über eine Signalleitung 33 mit einer über eine Leitung 51 mit elektrischer Energie aus einem hier nicht näher dargestellten elektrischen Versorgungsnetz gespeisten zentralen Steuerungseinheit 52 verbunden ist und welches die durchströmende Wassermenge mitsamt dem eingespeisten Entkeimungsmittel erfaßt und der zentralen Steuerungseinheit 52 übermittelt. Hierbei ist zu berücksichtigen, daß die Zugabemenge des Entkeimungsmittels für die Bestimmung der Zulaufmenge an Wasser in der Größenordnung von 1 bis 2 % liegt und damit vernachlässigbar gering ist.

Darüber hinaus kann durch programmtechnische Anpassung der Kennlinie beziehungsweise durch entsprechende Ausgleichsrechnung diese geringe Zugabemenge an Entkeimungsmittel berücksichtigt werden.

Ferner kann schaltungstechnisch vorgesehen sein, daß vor jeder Meßwerterfassung ein kurzfristiges Schließen des als Proportionalventil ausgebildeten Zulaufventils 22, dessen Verhalten ähnlich einem Kondensator ist, für wenige Millisekunden vorgesehen ist, um dieses vor der erneuten Ansteuerung mit der zutreffenden Spannung zu entladen. Hierdurch ist eine Drift des Istwertes ausgeschlossen. Da die Entnahme von behandeltem Wasser jeweils nur kurzzeitig erfolgt und die Zugabe von Entkeimungsmittel dabei diesem Intervallbetrieb folgt, kann eine eventuell auftretende driftbedingte geringe Istwertabweichung ebenfalls vernachlässigt werden.

Zwischen dem Durchflußmeßgerät 32 und der Anschlußstelle 30 für den oder die Verbraucher ist eine weitere Einleitungsstelle 34 für Entkeimungsmittel vorgesehen. Diese Einleitungsstelle 34 ist über eine zweite Zulaufleitung 36, in welcher ein zweites, ebenfalls elektrisch betätigbares Zulaufventil 38 angeordnet ist, mit der Zulaufleitung 20 verbunden.

Die Wirkungsweise dieser Dosiervorrichtung 10 ist wie folgt. Der Vorratsbehälter 12 wird über den Einfüllstutzen 14 mit Entkeimungsmittel befüllt. Das darin integrierte Sieb dient dazu, das Eindringen von Verunreinigungen in die Dosiereinrichtung zu verhindern. Um die Befüllung mit Entkeimungsmittel zu ermöglichen, ist die erwähnte Druckluftzufuhr über den Stutzen 16 beziehungsweise über die daran anschließende Leitung 17 mittels eines entsprechend betätigten Absperrorgans 40, Ventil oder Schieber, abgesperrt und der Vorratsbehälter 12 drucklos.

Sobald der Vorratsbehälter 12 ausreichend mit Entkeimungsmittel befüllt ist, wird der Einfüllstutzen 14 druckdicht verschlossen. Nun wird das Druckluftventil 40 geöffnet, so daß Druckluft aus der Leitung 17 über den Druckluftstutzen 16 in den Vorratsbehälter 12 gelangt und das darin befindliche Entkeimungsmittel beaufschlagt. Ein in der Druckluftleitung 17 angeordneter Druckregler 42 regelt den Zulaufdruck von etwa 0,4 - 0,5 MPa auf etwa zum Beispiel 0,3 MPa und sorgt so für gleichbleibend hohen Überdruck im Vorratsbehälter 12 sowie dafür, daß der Vorratsbehälter 12 keine unzulässige Beanspruchung durch zu hohen Überdruck erfährt. Ein ebenfalls vorgesehenes Manometer 44 zur Kontrolle zeigt den aktuellen Druckwert an. Hierbei ist zu beachten, daß für eine einwandfreie Funktion der Druckwert im Vorratsbehälter 12 stets ausreichend oberhalb des Wasserdruckes liegen muß.

Über den nahe dem Boden stirnseitig angeordneten Entnahmestutzen 18 gelangt das Entkeimungsmittel über die Zulaufleitung 20 zunächst zu einem darin angeordneten Feinsieb 46, welches eventuelle Verunreinigungen und/oder Verklumpungen des Entkeimungsmittels, die die Löslichkeit im Wasser beeinträchtigen können, zurückhält. Stromab folgt ein Rückschlagventil 48, welches eine Strömungsumkehr in der Leitung 20 und damit das Eindringen von Wasser in den Vorratsbehälter verhindert.

Hinter dem Rückschlagventil 48 ist in Strömungsrichtung eine Verzweigung 50 angeordnet. Von hier führt die erste Zulaufleitung 23 zum ersten Zulaufventil 22 und die zweite Zulaufleitung 36 zum zweiten Zulaufventil 38.

Entsprechend dem Wert der vom Durchflußmeßgerät 32 erfaßten Durchflußmenge wird ein Signal an die zentrale Steuerungseinheit 52 geleitet, welche dieses Signal verarbeitet und hieraus abgeleitet die Öffnung, das heißt die Öffnungsweite, des ersten Zulaufventils 22 vorgibt und dieses über eine Steuerleitung 21 ansteuert.

Gleichermaßen wird parallel hierzu über eine Steuerleitung 25 das Wasserventil 26 angesteuert. Ebenso wird über eine Signalleitung 13 der Füllstand des Vorratsbehälters 12 an die zentrale Steuerungseinheit 52 übermittelt. Die zentrale Steuerungseinheit 52 dient also sowohl zur Überwachung und Erfassung aller relevanten Betriebparameter als auch für die verfahrensoptimierte Ansteuerung der einzelnen Ventile.

Im Falle, daß eine besonders große Menge an Entkeimungsmittel einzuspeisen ist, zum Beispiel zur Grundimpfung, kann über die zweite Zulaufleitung 36 zusätzlich Entkeimungsmittel durch das zweite, über eine Steuerleitung 39 mit der zentralen Steuerungseinheit 52 verbundenen Zulaufventil 34 in die Wasserleitung 24 eingeleitet werden. Dies kann auch kombiniert sein mit einer Absperrung der Wasserleitung 24 durch das Ventil 26.

Die Zugabe des Entkeimungsmittels erfolgt, wie zuvor erläutert, vor dem Durchflußmesser, um so eine bessere Durchmischung des Entkeimungsmittels mit dem zu entkeimenden Wasser zu erreichen. Darüber hinaus können so die gegebenenfalls dem Entkeimungsmittel beigefügten Additive, die zum Beispiel die Ablagerung von Kalk und Algen verhindern sowie zusätzlich die Oberflächenspannung herabsetzen, besser ihre Wirkung entfalten und so den für möglichst lange Zeit störungsfreien Betrieb des Durchflußmessers sichern.

Zur Selbstkalibrierung sperrt die Steuerungseinrichtung 52 das Wasserventil 26. Hierdurch steht zwischen dem Vorratsbehälter 12 und der Anschlußstelle 30 der im Vorratsbehälter 12 herrschende Druck als Druckgefälle zur Verfügung, um das Entkeimungsmittel beim Öffnen eines Verbrauchers mit erhöhtem Durchfluß durch das Ventil 26 und den Durchflußmesser 32 ausfließen zu lassen.

Die Steuereinrichtung 52 gibt nacheinander zumindest drei festgelegte, verschiedene Spannungswerte an das Ventil 26 ab, das entsprechend öffnet. Über das Durchflußmeßgerät 32 erfaßt die Steuerungseinrichtung 52 die tatsächliche Durchflußmenge, errechnet die Abweichungen von der vorgegebenen Kennlinie (Durchflußmenge in Abhängigkeit der Ventilspannung) und bestimmt Korrekturwerte für eine Verschiebung der Kennlinie. Diese Korrekturwerte werden gespeichert (EEPROM) und stehen bis zum nächsten Kalibriervorgang zur Verfügung, um die aktuellen Sollwerte für die Spannungsvorgabe für das Ventil 26 zu errechnen.

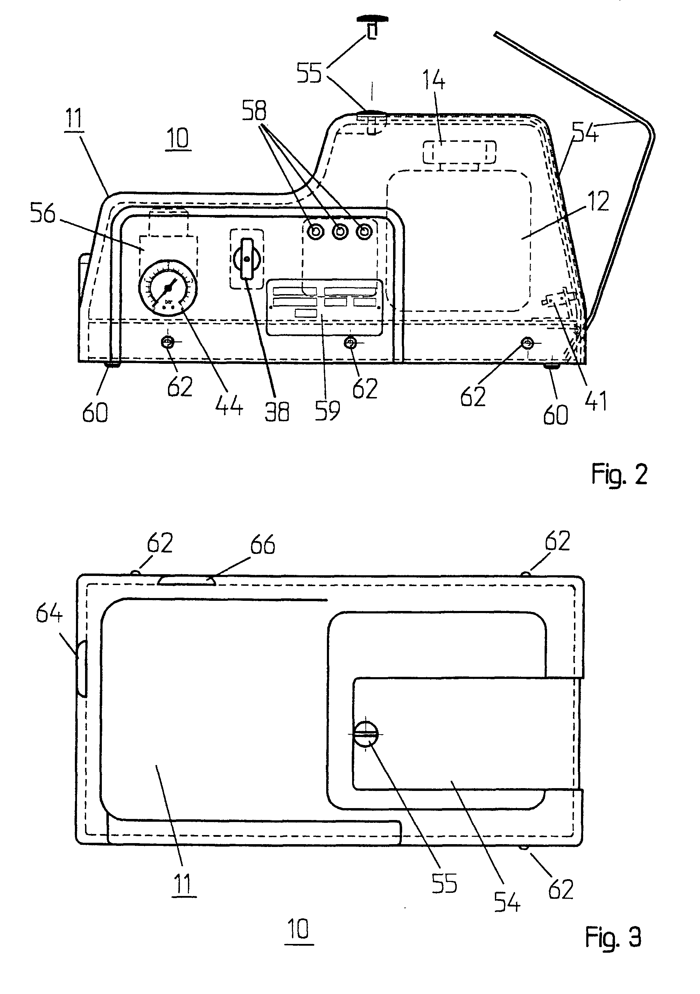

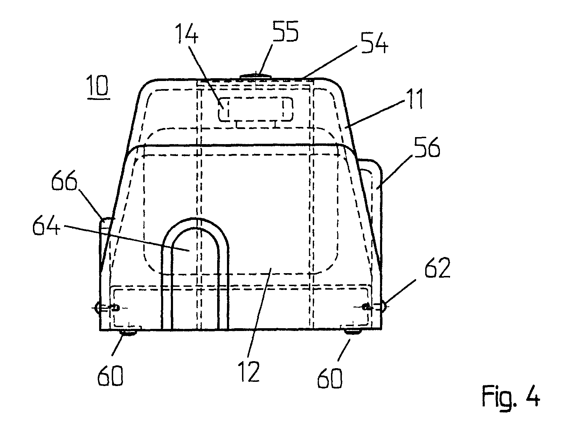

In Fig. 2 ist ein praktisch ausgeführtes Gerät einer erfindungsgemäßen Dosiervorrichtung 10 in Seitenansicht dargestellt, aus welcher Darstellung dessen sehr kompakte und gleichzeitig sehr zweckmäßige Gestaltung erkennbar ist. Die Darstellungen in den weiteren Figuren 3 und 4 verdeutlichen die Kompaktform des Gehäuses 11.

Das erfindungsgemäße Gerät 10 besitzt ein Gehäuse 11, dessen Wandung vorzugsweise aus einem pflegeleicht zu reinigenden antistatischen Kunststoff, zum Beispiel Polyurethan, besteht und deren Querschnitt anhand von die Innenseite anzeigenden gestrichelten Linien erkennbar ist. Das Gehäuse 11 ist stufenförmig ausgebildet und besitzt einen flachen Bereich und einen erhöhten Bereich. Der erhöhte Bereich, der in Fig. 2 auf der rechten Seite des Gehäuses 11 angeordnet ist, ist von einem schwenkbar an der unteren rechten Stirnseite des Gehäuses 11 angelenkten Klappdeckel 54 abgedeckt, der mittels eines Schraubverschlusses 55 in Ruhelage gehalten ist und der es auf einfache Weise ermöglicht, rasch zu dem im Inneren des Gehäuses 11 angeordneten Vorratsbehälter 12 zu gelangen, zum Beispiel um ihn aufzufüllen.

In Fig. 2 ist der Vorratsbehälter 12 mit gestrichelten Linien dargestellt, da er in dieser Ansicht durch die Seitenwand verdeckt und daher normalerweise nicht zu sehen ist. Ebenfalls gestrichelt dargestellt ist der oberhalb des Vorratsbehälters 12 angeordnete Einfüllstutzen mit Filter 14. Vom Klappdeckel 54 verdeckt ist an der rechten Stirnseite des Gehäuses 11 ein Entlüftungsventil 41 positioniert, das vorzugsweise in das Druckluftventil 40 integriert ist.

Unterhalb davon an der frontseitigen Seitenfläche des flachen Bereiches des Gehäuses 11, das heißt in der Zeichnungsebene, sind die Bedienungselemente zur Einschaltung des Gerätes beziehungsweise zur Einstellung der Betriebsparameter leicht zugänglich und übersichtlich auf einem Bedienpaneel 56 angeordnet.

Im einzelnen sind am Bedienpaneel 56 das Manometer 44 und das zweite Zulaufventil 38 angeordnet, das hier als manuell betätigbares Magnetventil gezeigt ist, sowie drei Leuchtanzeigen 58, die als vorzugsweise als LED ausgebildet sind. Schließlich ist auch ein Typenschild 59 am Paneel 56 angebracht.

An der Unterseite des Gehäuses 11 sind Sockelfüße 60, vorzugsweise aus Gummi, vorgesehen, auf welchen das Gerät weitgehend schallgedämpft aufgestellt ist. Ferner sind an den Seiten des Gehäuses 11 Fixierschrauben 62 zur Befestigung des Gehäuses 11 vorgesehen, mittels derer eine Befestigung innerhalb einer größeren Anlage möglich ist.

An der dem Klappdeckel 54 gegenüberliegenden Stirnseite, die in Fig. 4 als Seitenansicht gezeigt ist, sowie an der dem Bedienpaneel 56 gegenüberliegenden Seite des Gehäuses 11 sind je eine besondere Öffnung 64, 66 als sogenannter Schlauchausgang angeordnet, durch welche die Zulaufleitungen 17, 19 für die Druckluft sowie für das Wasser geführt sind.

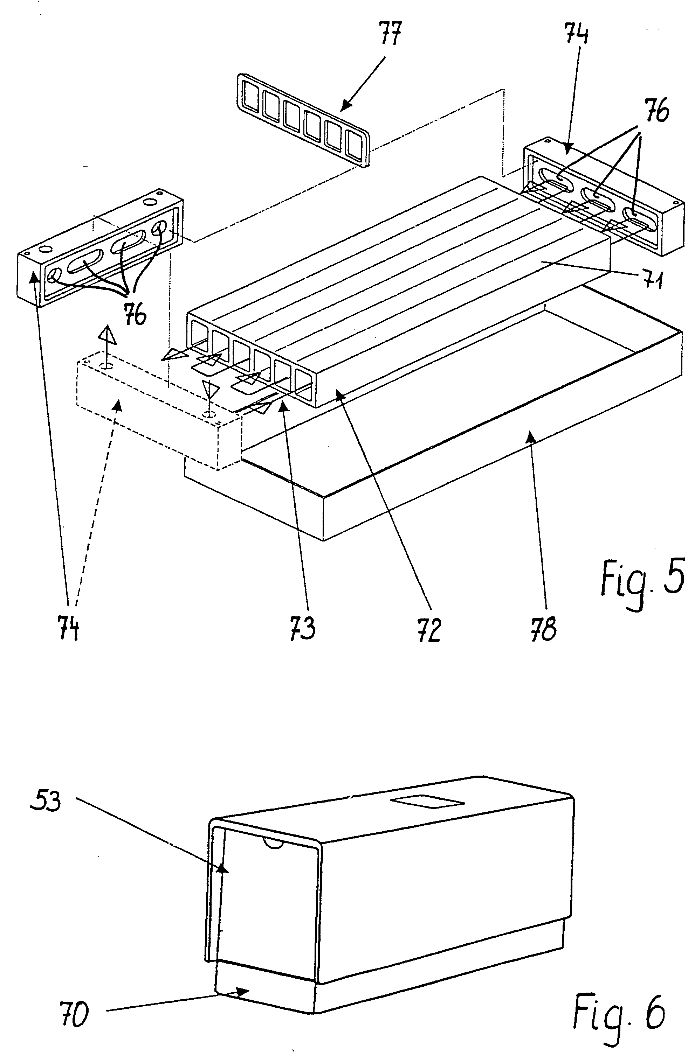

In Fig. 5 ist der Aufbau eines Pufferspeichers 70 dargestellt. Er besteht aus einem flachen Rohrpaket 72, welches von mehreren, aneinanderliegenden Rohren 71 mit quadratischem Querschnitt gebildet ist. Zur Bildung eines durchgehenden Strömungspfades 73 sind stirnseitig Endstücke 74 vorgesehen, die jeweils Aushöhlungen 76 derart aufweisen, daß eine fluiddichte, paarweise Verbindung zwischen jeweils zwei benachbarten Rohren 71 gegeben ist. Zwischen den Endstücken 74 und dem Rohrpaket 70 sind Dichtungen 77 vorhanden.

Der so gebildete Pufferspeicher 70 ist in ein Aufnahmegehäuse 78 eingesetzt, das unmittelbar am Gehäuse 53 der Dosiervorrichtung anbringbar ist, wie in Fig. 6 dargestellt. Ebenso ist es auch möglich, bei entsprechend anders gestalteter Ausbildung des Gehäuses 78 den Pufferspeicher 70 der Dosiervorrichtung nachgeschaltet in den Strömungsweg einzusetzen.

### Bezugszeichenliste

- 10: Dosiervorrichtung
- 12: Vorratsbehälter
- 13: Signalleitung (Füllstand)
- 14: Einfüllstutzen mit Filter
- 16: Druckluftstutzen
- 17: Druckluftleitung
- 18: Auslaßstutzen
- 19: Wasserspeiseleitung

- 20: Zulaufleitung
- 21: Steuerleitung (erstes Zulaufventil)
- 22: erstes Zulaufventil
- 23: erste Zulaufleitung
- 24: Wasserleitung, Strömungsweg
- 25: Steuerleitung (Wasserventil)
- 26: Wasserventil
- 28: erste Einleitungsstelle

- 30: Anschlußstelle
- 32: Durchflußmeßqerät
- 33: Signalleitung
- 34: zweite Einleitungsstelle
- 36: zweite Zulaufleitung
- 38: zweites Zulaufventil
- 39: Steuerleitung (zweites Zulaufventil)

- 40: Druckluftventil
- 41: Entlüftungsventil
- 42: Druckluftregler
- 44: Manometer
- 46: Feinsieb
- 48: Rückschlagventil

- 50: Verzweigung
- 51: elektrische Versorgungsleitung
- 52: Steuereinrichtung
- 53: Gehäuse
- 54: Klappdeckel
- 55: Schraubverschluß
- 56: Paneel
- 58: Leuchtanzeigen (LED)
- 59: Typenschild

- 60: Sockelfüße
- 62: Befestigungsschrauben
- 64: Schlauchdurchführung
- 66: Schlauchdurchführung

- 70: Pufferspeicher
- 71: Rohr
- 72: Rohrpaket
- 73: Strömungspfad
- 74: Endstück
- 76: Aushöhlungen
- 77: Dichtung
- 78: Aufnahmegehäuse

## Patentansprüche

1. Dosiervorrichtung zur Zugabe von Entkeimungs- oder Desinfektionsmittel in eine wassergespeiste Versorgungseinrichtung zur Bereitstellung von entkeimtem Wasser für Verbraucher, mit
- einem Vorratsbehälter (12) für Entkeimungs- oder Desinfektionsmittel,
- einem Strömungsweg (24) für das Wasser, welches wenigstens einer Anschlußstelle (30) zuströmt,
- einer fernbetätigbaren Zugabeeinrichtung (22) für das Entkeimungsmittel,
- einer Steuereinrichtung (52),
die mit einem Durchflußmesser zusammenarbeitet, welcher die Durchflußmenge des eingespeisten Wassers erfaßt und als Signal an die Steuerungseinrichtung (52) überträgt, und die die Zugabeeinrichtung (22) in Abhängigkeit einer vorgebbaren Konzentration von Entkeimungsmittel im Wasser betätigt,
dadurch gekennzeichnet, daß
- der Vorratsbehälter (12) mit Druckluft beaufschlagbar ist, und
- die Zugabeeinrichtung als von der Steuerungseinrichtung (52) ansteuerbares Proportionalventil (22) ausgebildet ist, das in einen Zulauleitung (23) zum Strömungsweg des Wassens angeordnet ist.

2. Dosiervorrichtung nach Anspruch 1, dadurch gekennzeichnet, daß das Proportionalventil (22) ein Piezoventil ist.

3. Dosiervorrichtung nach Anspruch 1 oder 2, dadurch gekennzeichnet, daß die Konzentration variabel, vorzugsweise zwischen 0 % und 100 %, einstellbar ist.

4. Dosiervorrichtung nach einem der vorherigen Ansprüche, gekennzeichnet durch eine Einrichtung zur Erfassung des IST-Wertes der Konzentration, die mit der Steuereinrichtung (52) zu einem geschlossenen Regelkreis verbunden ist.

5. Dosiervorrichtung nach einem der vorherigen Ansprüche, gekennzeichnet durch eine Einrichtung zur Selbstkalibrierung.

6. Dosiervorrichtung nach einem der vorherigen Ansprüche, dadurch gekennzeichnet, daß die Einleitung des Entkeimungsmittels in den Strömungsweg (24) des eingespeisten Wassers vor dem Durchflußmesser (32) vorgesehen ist.

7. Dosiervorrichtung nach einem der vorherigen Ansprüche, dadurch gekennzeichnet, daß der Durchflußmesser (32) wenigstens einen Drehkörper aufweist, insbesondere ein Flügelrad, welcher das eingespeiste Wasser und das eingeleitete Entkeimungsmittel durcheinanderwirbelt und hierdurch innig miteinander vermischt.

8. Dosiervorrichtung nach einem der vorherigen Ansprüche, dadurch gekennzeichnet, daß die Stelle (28) der Einleitung des Entkeimungsmittels in den Strömungsweg (24) des eingespeisten Wassers nahe der Anschlußstelle (30) vorgesehen ist.

9. Dosiervorrichtung nach einem der vorherigen Ansprüche, dadurch gekennzeichnet, daß zwischen der Stelle (28) der Einleitung des Entkeimungsmittels in den Strömungsweg (24) des Wassers und dem Vorratsbehälter (12) ein Rückschlagventil (48) angeordnet ist, welches den Zutritt von Wasser in den Vorratsbehälter (12) sicher verhindert.

10. Dosiervorrichtung nach einem der vorherigen Ansprüche, dadurch gekennzeichnet, daß zwischen der Stelle (28) der Einleitung des Entkeimungsmittels in den Strömungsweg (24) des Wassers und dem Vorratsbehälter (12) eine Filtereinrichtung (46) angeordnet ist, welches den Einleitung von Verunreinigungen aus dem Vorratsbehälter sicher ausschließt.

11. Dosiervorrichtung nach einem der vorherigen Ansprüche, dadurch gekennzeichnet, daß für die Einleitung des Entkeimungsmittels in den Strömungsweg (24) des Wassers eine zweite Zulaufleitung (36) mit einem zweiten Zulaufventil (38) vorgesehen ist, welches parallel zur ersten arbeitet und für eine raschere Einleitung des Entkeimungsmittels sorgt.

12. Dosiervorrichtung nach einem der vorherigen Ansprüche, gekennzeichnet durch einen nachgeschalteten Pufferspeicher (70) für entkeimtes Wasser.

13. Dosiervorrichtung nach Anspruch 12, dadurch gekennzeichnet, daß der Pufferspeicher (70) aus einem Rohrpaket (72) aufgebaut ist, das zur Bildung eines durchgehenden Strömungspfads (73) jeweils stirnseitig mit Endstücken (74) versehen ist, wobei die Endstücke (74) Aushöhlungen (76) aufweisen, die eine fluiddichte, paarweise Verbindung zwischen jeweils zwei benachbarten Rohren (71) bilden.

14. Dosiervorrichtung nach Anspruch 12 oder 13, dadurch gekennzeichnet, daß der Pufferspeicher (70) in ein Aufnahmegehäuse (78) eingesetzt und als Zusatzmodul ausgebildet ist, welches unmittelbar am Gehäuse (53) der Dosiervorrichtung oder der Dosiervorrichtung nachgeschaltet anbringbar ist.

15. Dosiervorrichtung nach einem der vorherigen Ansprüche, dadurch gekennzeichnet, daß sie als Zusatzmodul ausgebildet ist, welches nachträglich in eine bestehende Geräteanordnung für die Dentalhygiene integrierbar ist.

16. Verwendung einer Dosiervorrichtung nach einem der vorherigen Ansprüche zur Bereitstellung von keimfreiem Wasser.

17. Verwendung einer Dosiervorrichtung nach einem der Ansprüche 1-15 für Einrichtungen der Dentalhygiene.

## Claims

1. Dosing device for addition of germicidal or disinfecting material in a water supplied supply device for preparing sterilized water for users, with
- a supply container (12) for germicide or disinfectant,
- a flow path (24) for water, which flows to at least one junction point (30),
- a remote operable supply device (22) for the germicide,
- a control unit (52),
which works in cooperation with a flow meter, which detects the flow volume of the supplied water and transmits this as a signal to the control unit (52), and which controls the supply device (22) to introduce germicide into the water at a predetermined concentration,
thereby characterized, that
- the supply container (12) can be charged with pressurized air, and
- the supply device is formed as proportional valve (22) controllable by the control unit (52), which is provided in a supply line (23) leading to the water flow path.

2. Dosing device according to Claim 1, thereby characterized, that the proportional valve (22) is a piezoelectric valve.

3. Dosing device according to Claim 1 or 2, thereby characterized, that the concentration is variably adjustable, preferably between 0% and 100%.

4. Dosing device according to one of the preceding claims, characterized by a device for sensing the actual value of the concentration, which is connected to the control unit (52) in a closed feed back loop.

5. Dosing device according to one of the preceding claims, characterized by a device for self calibration.

6. Dosing device according to one of the preceding claims, thereby characterized, that the point of introduction of the germicide into the flow path (24) of the supplied water is upstream of the flow meter (32).

7. Dosing device according to one of the preceding claims, thereby characterized, that the flow meter (32) is comprised of at least one rotational body, in particular a propeller, which stirs the supplied water and the introduced germicide and thereby mixes these thoroughly with each other.

8. Dosing device according to one of the preceding claims, thereby characterized, that the point (28) of the introduction of the germicide into the flow path (24) of the supply water is close to the connection point (30).

9. Dosing device according to one of the preceding claims, thereby characterized, that between the point (28) of the introduction of the germicide into the flow path (24) of the water and the supply container (12) a back flow valve (48) is provided, which prevents entry of water into the supply container (12).

10. Dosing device according to one of the preceding claims, thereby characterized, that between the point (28) of the introduction of the germicide into the flow path (24) of the water and the supply container (12) a filtering device (46) is provided which prevents introduction of impurities from the supply chamber.

11. Dosing device according to one of the preceding claims, thereby characterized, that for the introduction of the germicide into the flow path (24) of the water a second supply line (36) with a second supply valve (38) is provided, which operates parallel to the first and makes possible a more rapid introduction of the germicide.

12. Dosing device according to one of the preceding claims, characterized by a downstream connected buffer storage (70) for disinfected water.

13. Dosing device according to Claim 12, thereby characterized, that the buffer storage (70) is comprised of a tube packet (72), which for the formation of a through going flow path (73) is provided respectively with end pieces (74), wherein the end pieces (74) exhibit through holes (76) which form a fluid tight pair-wise connection between respectively adjacent pipes (71).

14. Dosing device according to Claim 12 or 13, thereby characterized, that the buffer storage (70) is seated or introduced in a receptacle housing (78) and is formed as a supplemental module, which can be provided immediately adjacent to the housing (53) of the dosing device or connected downstream of the dosing device.

15. Dosing device according to one of the preceding claims, thereby characterized, that it is constructed as a add-on module, which can be retrofitted into an existing apparatus for dental hygiene.

16. Use of a dosing device according to one of the preceding claims for providing sanitized water.

17. Use of a dosing device according to one of claims 1-15, as an apparatus for dental hygiene.

## Revendications

1. Dispositif de dosage pour délivrer un agent germicide ou désinfectant dans un dispositif d'alimentation recevant de l'eau pour fournir de l'eau sans germes à des consommateurs, comprenant :
- un réservoir d'alimentation (12) pour l'agent germicide ou désinfectant,
- un chemin de circulation (24) pour l'eau qui arrive à au moins un point de raccordement (30),
- une installation d'addition (22) télécommandée pour l'agent germicide,
- une installation de commande (52) coopérant avec un débitmètre qui saisit le débit de l'eau introduite et le transmet comme signal à l'installation de commande (52), et qui actionne l'installation d'addition (22) en fonction d'une concentration prédéterminée d'agent germicide dans l'eau,
caractérisé en ce que
- le réservoir d'alimentation (12) peut recevoir de l'air comprimé, et
- l'installation d'addition est formée par une vanne proportionnelle (22) commandée par l'installation de commande (52), et placée dans une conduite d'alimentation (23) alimentant le chemin de circulation de l'eau.

2. Dispositif de dosage selon la revendication 1,
caractérisé en ce que
la vanne proportionnelle (22) est une vanne piézo-électrique.

3. Dispositif de dosage selon l'une quelconque des revendications 1 ou 2,
caractérisé en ce que
la concentration est variable et réglable de préférence entre 0 % et 100 %.

4. Dispositif de dosage selon l'une quelconque des revendications précédentes,
caractérisé par
une installation destinée à saisir la valeur réelle de la concentration, et reliée à l'installation de commande (52) pour former un circuit de régulation fermé.

5. Dispositif de dosage selon l'une quelconque des revendications précédentes,
caractérisé par
une installation d'autocalibrage.

6. Dispositif de dosage selon l'une quelconque des revendications précédentes,
caractérisé en ce que
l'introduction de l'agent germicide dans le chemin de circulation (24) de l'eau d'alimentation est prévue en amont du débitmètre (32).

7. Dispositif de dosage selon l'une quelconque des revendications précédentes,
caractérisé en ce que
le débitmètre (32) comporte au moins un organe tournant, notamment un rotor à ailette qui mélange l'eau d'alimentation et l'agent germicide introduit pour en assurer le mélange intime.

8. Dispositif de dosage selon l'une quelconque des revendications précédentes,
caractérisé en ce que
le point (28) d'introduction de l'agent germicide dans le chemin de circulation (24) de l'eau d'alimentation est à proximité du point de raccordement (30).

9. Dispositif de dosage selon l'une quelconque des revendications précédentes,
caractérisé en ce qu'
entre le point (28) d'introduction de l'agent germicide dans le chemin de circulation (24) de l'eau et le réservoir d'alimentation (12) il est prévu un clapet anti-retour (48) qui interdit en toute sécurité l'arrivée d'eau dans le réservoir d'alimentation (12).

10. Dispositif de dosage selon l'une quelconque des revendications précédentes,
caractérisé en ce qu'
entre le point (28) d'introduction de l'agent germicide dans le chemin de circulation (24) de l'eau et le réservoir d'alimentation (12) il est prévu une installation de filtre (46) qui interdit l'introduction d'impuretés à partir du réservoir d'alimentation.

11. Dispositif de dosage selon l'une quelconque des revendications précédentes,
caractérisé en ce que
pour l'introduction de l'agent germicide dans le chemin de circulation (24) de l'eau il est prévu une seconde conduite d'alimentation (36) avec une seconde vanne d'alimentation (38) travaillant en parallèle avec la première et permettant une entrée rapide de l'agent germicide.

12. Dispositif de dosage selon l'une quelconque des revendications précédentes,
caractérisé par
un réservoir tampon (70) installé en aval pour recevoir l'eau purifiée.

13. Dispositif de dosage selon la revendication 12,
caractérisé en ce que
le réservoir tampon (70) est formé d'un paquet de tubes (72) muni chaque fois du côté frontal d'embouts (74) pour former un chemin de passage continu (73),
les embouts (74) ayant des cavités (76) qui forment une liaison étanche au fluide, par paires, entre chaque fois deux tubes voisins (71).

14. Dispositif de dosage selon l'une quelconque des revendications 12 ou 13,
caractérisé en ce que
le réservoir tampon (70) est placé dans un boîtier (78) et est réalisé en forme de module complémentaire qui est mis directement sur le boîtier (53) du dispositif de dosage ou en aval du dispositif de dosage.

15. Dispositif de dosage selon l'une quelconque des revendications précédentes,
caractérisé en ce qu'
il est réalisé sous la forme de module accessoire qui s'intègre dans un dispositif existant pour l'hygiène dentaire.

16. Application d'un dispositif de dosage selon l'une quelconque des revendications précédentes pour fournir de l'eau sans germes.

17. Application d'un dispositif de dosage selon l'une quelconque des revendications 1 à 15 pour des installations d'hygiène dentaire.
